(19) Europäisches Patentamt / European Patent Office / Office européen des brevets

(11) **EP 4 076 281 B1**

(12) **EUROPEAN PATENT SPECIFICATION**

(45) Date of publication and mention
of the grant of the patent:
**29.04.2026  Bulletin 2026/18**

(21) Application number: **20903422.2**

(22) Date of filing: **17.12.2020**

(51) International Patent Classification (IPC):
*A61F 2/16* (2006.01)     *A61F 2/14* (2006.01)
*G02B 5/18* (2006.01)     *G02C 7/04* (2006.01)
*G02C 7/08* (2006.01)

(52) Cooperative Patent Classification (CPC):
**A61F 2/1637; A61F 2/1616; G02B 27/0075;**
G02B 3/0056; G02C 7/022; G02C 7/04

(86) International application number:
**PCT/US2020/065557**

(87) International publication number:
**WO 2021/127148 (24.06.2021 Gazette 2021/25)**

(54) **HIGH DEFINITION AND EXTENDED DEPTH OF FIELD INTRAOCULAR LENS**

HOHE AUFLÖSUNG UND ERWEITERTE SCHÄRFENTIEFE FÜR INTRAOKULARE LINSE

LENTILLE INTRAOCULAIRE DE HAUTE DÉFINITION À PROFONDEUR DE CHAMP ÉTENDUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **20.12.2019   US 201962951839 P
20.12.2019   US 201962951832 P
24.07.2020   US 202063056110 P**

(43) Date of publication of application:
**26.10.2022   Bulletin 2022/43**

(73) Proprietor: **Z Optics, Inc.
Cookeville, TN 38501 (US)**

(72) Inventors:
• **SARVER, Edwin, J.**
**Cookeville, TN 38501 (US)**
• **SIMMS, James, J.**
**Medford, NJ 08055 (US)**

(74) Representative: **HGF**
**HGF Europe LLP**
**Neumarkter Straße 18**
**81673 München (DE)**

(56) References cited:
**WO-A1-2010/100523     WO-A1-2018/076057
US-A1- 2005 068 494     US-A1- 2008 297 720
US-A1- 2018 275 427     US-A1- 2019 307 556
US-B2- 8 747 466     US-B2- 8 974 526
US-B2- 9 636 215**

Description

## CROSS-REFERENCE TO RELATED APPLICATIONS

This application claims priority to U.S. Patent Application No. 62/951,832, filed December 20, 2019, entitled "HIGH DEFINITION AND EXTENDED DEPTH OF FIELD INTRAOCULAR LENS WITH HEXAGONAL SAMPLED MICRO-LENSES", U.S. Patent Application No. 62/951,839, filed December 20, 2019, entitled "HIGH DEFINITION AND EXTENDED DEPTH OF FIELD INTRAOCULAR LENS WITH MULTI-FOCAL OPTICAL ZONE", and U.S. Patent Application No. 63/056,110, filed July 24, 2020, entitled "HIGH DEFINITION AND EXTENDED DEPTH OF FIELD INTRAOCULAR LENS".

## BACKGROUND

The human eye often suffers from aberrations such as defocus and astigmatism that must be corrected to provide acceptable vision to maintain a high quality of life. Correction of these defocus and astigmatism aberrations can be accomplished using a lens. The lens can be located, for example, at a spectacle plane, at the corneal plane (a contact lens or corneal implant), or within the eye as a phakic (crystalline lens intact) or aphakic (crystalline lens removed) intraocular lens (IOL). Prior patent document describes an example of an ophthalmic lens for comprising lenslets for preventing and/or slowing myopia progression.

In addition to the basic aberrations of defocus and astigmatism, the eye often has higher-order aberrations such as spherical aberration and other aberrations. Chromatic aberrations, which are generally aberrations due to varying focus with wavelength across the visible spectrum, are also present in the eye. These higher-order aberrations and chromatic aberrations negatively affect the quality of a person's vision. The negative effects of the higher-order and chromatic aberrations increase as the pupil size increases. Vision with these aberrations removed is often referred to as high definition (HD) vision.

Presbyopia is the condition where the eye loses its ability to focus on objects at different distances. Aphakic eyes have presbyopia. A standard monofocal IOL implanted in an aphakic eye restores vision at a single focal distance. A variety of devices and procedures are used to provide improved vision over a range of distances, among them, using a monofocal IOL combined with bi-focal or progressive addition spectacles. A monovision IOL system is another option to restore near and distance vision - one eye is set at a different focal length than the fellow eye, thus providing binocular summation of the two focal points and providing blended visions. Monovision is currently the most common method of correcting presbyopia by using IOLs to correct the dominant eye for distance vision and the non-dominant eye for near vision in an attempt to achieve spectacle-free binocular vision from far to near.

Additionally, IOLs can be multifocal, for example, bifocal (having two focal regions - usually far and near) or trifocal (having three focal regions - far, intermediate, and near). Most multifocal IOLs are designed to have one or more focal regions distributed within an addition range. However, using elements with a set of discrete foci is not the only possible strategy of design: the use of elements with extended depth of field (EDOF), that is, elements producing a continuous focal segment spanning the required addition, can also be considered. These methods are not entirely acceptable as stray light from the various focal regions degrade a person's vision. US2005068494A1 discloses an IOL with a virtual aperture generated by a large radial optical power increase.

## SUMMARY

Disclosed are systems, devices, and methods that overcome limitations of IOLs at least by providing by providing a phakic or aphakic IOL that simultaneously provides correction of defocus and astigmatism, decreases higher-order and chromatic aberrations, and provides an extended depth of field to improve vision quality. In addition, a central optic of the IOL provides a small "add" sector to increase the quality of vision corresponding to objects in a "near vision" region.

In one aspect, there is disclosed an intraocular lens configured to provide an extended depth-of-field, said intraocular lens comprising: an optical zone comprising at least one anterior optical surface and at least one posterior optical surface; a first periphery region peripherally positioned relative to the optical zone, the first periphery region comprising a virtual aperture, the virtual aperture comprising an anterior virtual aperture surface and a posterior virtual aperture surface, wherein the virtual aperture comprises a plurality of hexagonal micro-structures, each hexagonal micro-structure comprising a micro-lens; and a second periphery region peripherally positioned relative to the first periphery region, the second periphery region comprising a haptic for positioning the intraocular lens within an eye, wherein the haptic comprises an outermost region of the intraocular lens; wherein a first plurality of light rays incident on the anterior optical surface pass through the optical zone to form an image on a retina when the intraocular lens is implanted in an eye; and wherein a second plurality of light rays incident on the anterior virtual aperture surface are dispersed widely downstream from the intraocular lens towards and across the retina, such that the image comprises the extended depth-of-field and further wherein said virtual aperture reduces monochromatic and chromatic aberrations in the image.

In a related method, an IOL, such as any embodiment of an IOL described herein, is implanted or otherwise coupled to an eye, such as a human eye. The IOL is used to modify or adjust a transmission of lights rays onto a retina of an eye pursuant to the features described herein.

The disclosed IOL has an optical configuration that allows central focused light to reach the central focal area of the retina and spread defocused and aberrated light to a periphery of the retina. In one or more IOL regions where defocused and aberrated light is widely spread across the retina, high power refractive and/or total internal reflection is employed. The result is an optical configuration that increases depth of focus and decrease monochromatic and chromatic aberrations providing high definition vision over a wide range of object distances from far to near vision.

The details of one or more variations of the subject matter described herein are set forth in the accompanying drawings and the description below.

## BRIEF DESCRIPTION OF THE DRAWINGS

Figs. 1A and 1B illustrates a basic method of reducing monochromatic aberrations and increasing or extending depth of field using pupil size for a near-sighted eye.

Figs. 2A and 2B illustrate a basic method of reducing monochromatic aberrations and increasing depth of field using pupil size for a far-sighted eye.

Figs. 3A and 3B illustrate a basic method of reducing monochromatic aberrations and increasing depth of field using pupil size for an emmetropic eye.

Figs. 4A and 4B illustrate the basic method of reducing chromatic aberrations using pupil size.

Figs. 5A and 5B illustrate the basic concept of the virtual aperture to limit the effective pupil size.

Figs. 6A, 6B, and 6C illustrates an overall structure of an example IOL.

Fig. 6D shows another embodiment of an example IOL.

Fig. 7 illustrates an IOL with hexagonal sampled micro-lenses in a virtual aperture zone in accordance with the present invention.

Fig. 8 illustrates an example hexagon geometry of a micro-lens.

Fig. 9 illustrates a center portion of a two-dimensional array of hexagons of micro-lenses.

Figs. 10A and 10B illustrate two neighboring hexagons, the neighboring corresponding micro-lens spheres, and a smooth surface profile that supports a minimum curvature.

Fig. 11 Illustrates an example partition of an optical zone of an IOL to provide both near and distance vision partitions.

## DETAILED DESCRIPTION

Before the present subject matter is further described, it is to be understood that this subject matter described herein is not limited to particular embodiments described, as such may of course vary. It is also to be understood that the terminology used herein is for the purpose of describing particular embodiments only, and is not intended to be limiting. Unless defined otherwise, all technical terms used herein have the same meaning as commonly understood by one skilled in the art to which this subject matter belongs.

Disclosed are systems, devices, and methods that overcome limitations of IOLs at least by providing a phakic or aphakic IOL that provides correction of defocus and astigmatism, decreases higher-order monochromatic and chromatic aberrations, and provides an extended depth of field to improve vision quality. The disclosed IOL is sometimes referred to herein as the Z+ optic or Z+ IOL. U.S. Patent No. 10,285,807 and U.S. Patent Application Serial No. 16/380,622 describe related systems and methods.

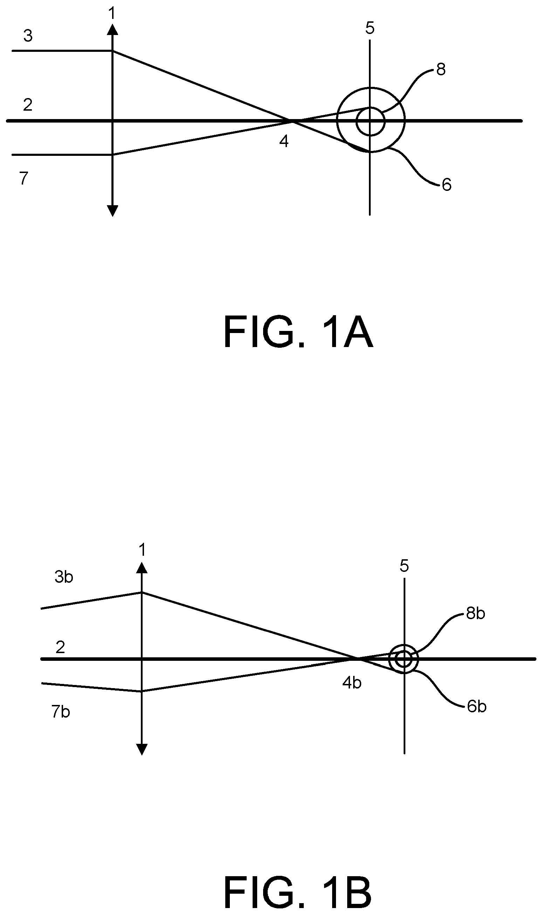

A description of the basic principle used to reduce monochromatic and chromatic aberrations and provide an increased depth of field is now provided. Figure 1A schematically illustrates a single converging lens 1 centered on an optical axis 2. An incident ray 3 from a distant object is parallel to the optical axis and intersects the focal point 4 (with a suffix b, c, d, e, or f based on the corresponding figure) of the lens. If the lens power is properly selected, the focal point coincides with the observation plane 5, otherwise there is a mismatch between the lens power and the location of the observation plane such that the focus is in front of or behind the observation plane.

In Figure 1A, the focal point is in front of the observation plane. If all incident rays are traced with the same ray height as incident ray 3, a blur circle 6 is located on the observation plane 5. The observation plane is oriented orthogonal to the optical axis and so is shown as a vertical line in the figure. The blur circles 6 and 8 are shown in the plane of the figure for visualization convenience, however, the blur circles are actually contained in the observation plane. Other parallel incident rays with ray height less than incident ray 3 fall inside this blur circle 6. One such ray is parallel incident ray 7 which is closer to the optical axis than incident ray 3. Incident ray 7 also intersects the focal point 4 and then the observation plane 5. Tracing all incident rays with ray height equal to incident ray 7 traces out blur circle 8 which has a diameter smaller than that of blur circle 6.

Figure 1B illustrates the same optical system in Figure 1A, but now the incident rays are for an object closer to the optical system as indicated by the slopes on incident rays 3b and 7b. The effect is that the focus point 4 (with a suffice a, b, c, d, or f based on the corresponding figure) for the closer object is now closer to the observation plane and both of the blur circles 6b and 8b are smaller than their counter parts in Figure 1A, but the principle is the same: rays which intersect the lens 1 closer to the optical axis have smaller blur on the observation plane. To relate this simple optical construction of Figure 1 to the human eye, the converging lens 1 represents the principal plane of the eye's optics including the cornea and the crystalline lens or an intraocular lens. Observation plane 5 represents the retina. As drawn the focal point 4 is in front of the observation plane (retina), so this figure is for a myopic or near-sighted eye. The size of the blur circles 6 and 8 (or 6b and 8b) represents the amount of defocus on the retina, where a smaller blur circle diameter provides clearer vision than a larger blur circle diameter.

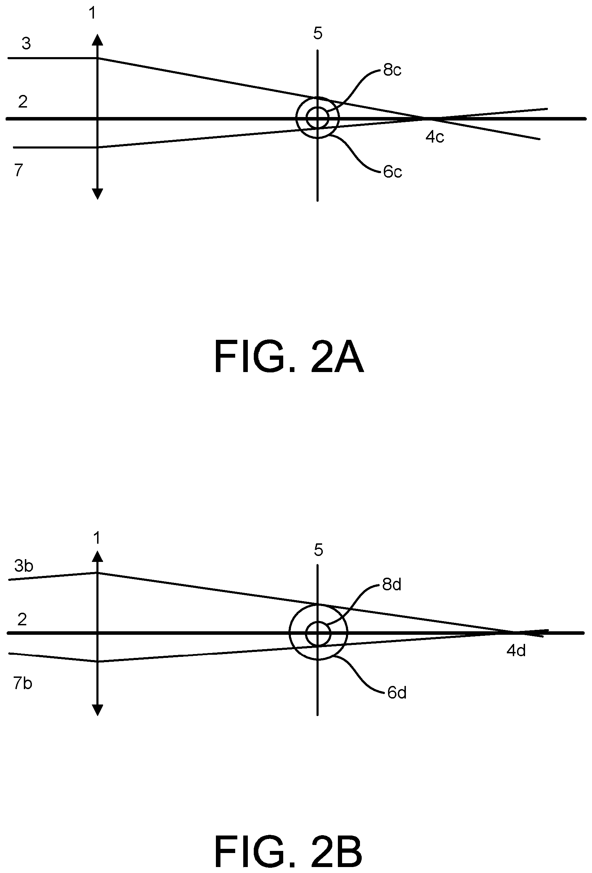

Note that the same relationship regarding incident ray height and blur circle size also holds for hyperopic or far-sighted eyes. This is schematically illustrated in Figures 2A and 2B, which show rays corresponding to a far-sighted eye. In Figure 2A for rays 3 and 7 from a distant object and in Figure 2B for rays 3b and 7b, smaller ray height leads to a smaller blur circle on the retina (observation plane).

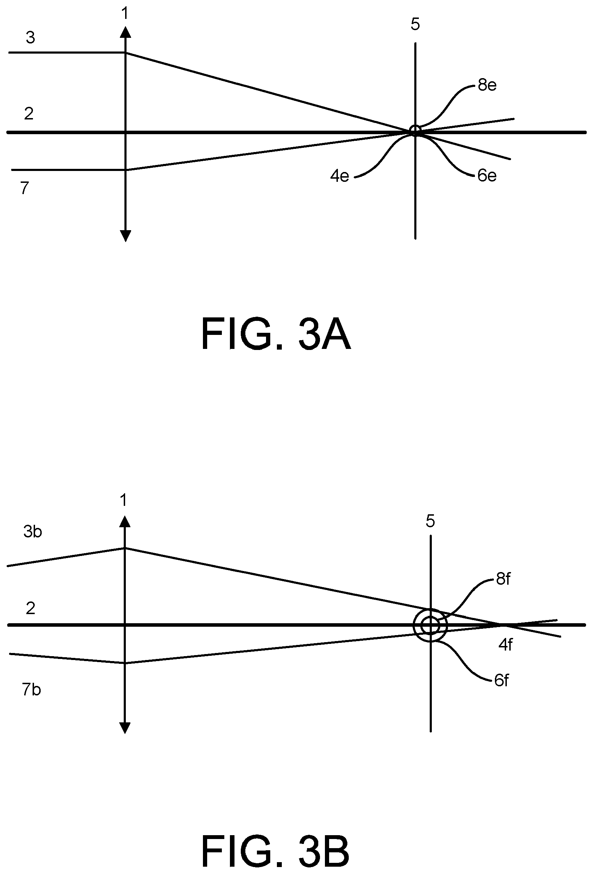

Similarly, Figures 3A and 3B (collectively referred to as Figure 3) show that the same parallel ray height to blur circle diameter property holds for an emmetropic eye. For a distant object, the focal point 4e is now at the retina (since the eye is emmetropic) and the blur circles 6e and 8e have zero radius. For a closer object, the focal point 4f is behind the retina and blur circle 8f corresponding to ray 7b which is closer to the optic axis has a smaller diameter than blur circle 6f corresponding to ray 3b which is further from the optic axis.

In general, an eye has aberrations, which means that as an incident ray location changes, the focal point in the eye also changes. But regardless of where the focal points are located (in front of-, on-, or behind the retina), as incident ray heights are reduced so are the blur circle diameters on the retina. Stated another way, for a given amount of defocus (dioptric error) in the eye, vision is improved as the height of incident rays is reduced. This principle is used when someone squints causing the eyelids to block the incident rays further from the optic axis of the eye in an attempt to see an out-of-focus distant or near object more clearly.

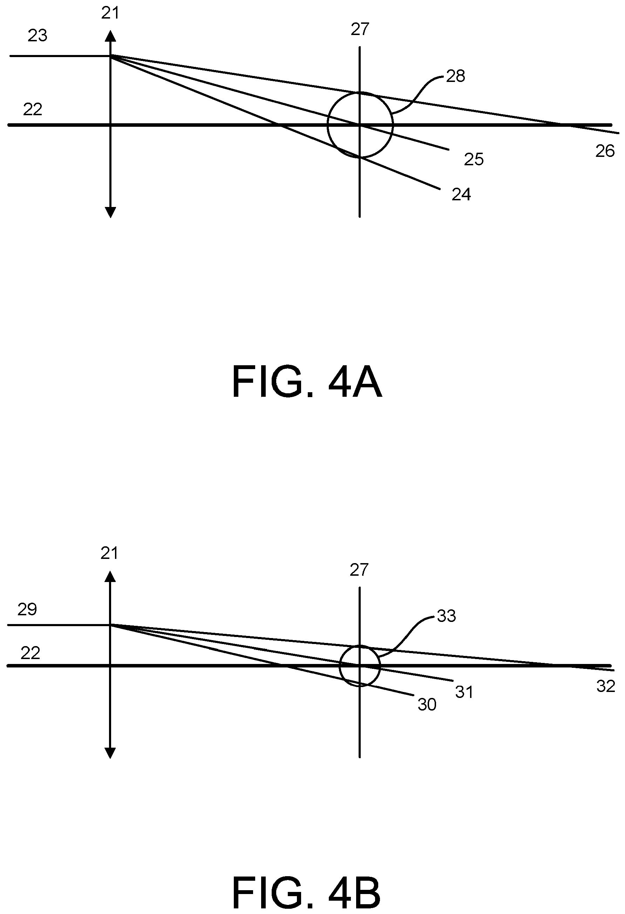

The ray tracing illustrated in Figures 1A-3B is for a single wavelength of incident light. For polychromatic light, multiple wavelengths are present. This is commonly illustrated by three rays of different wavelengths as shown in Figures 4A and 4B (collectively referred to as Figure 4). It is well known that for the components of the eye and typical optical materials, as the wavelength of light increases, the refractive index decreases.

In Figure 4A, a converging lens 21 has optical axis 22. An incident chromatic ray 23 consists of three wavelengths for blue (450 nm), green (550 nm), and red (650 nm) light which approximately span the range of visible light. Due to different indices of refraction for the three wavelengths, the blue light ray 24 is refracted more than the green light ray 25, and the green light ray is refracted more than the red light ray 26. If the green light ray is in focus, then it crosses the observation plane 27 at the optical axis. The chromatic spread of these three rays lead to a chromatic blur 28 on the observation plane.

In Figure 4B, the incident chromatic ray 29 has a lower ray height than the chromatic ray 23 in 4A. This leads to smaller chromatic blur 33 at the observation plane. Thus, just as for the monochromatic blur of Figures 1A-3B, chromatic blur is decreased as the chromatic ray height is decreased. The situation in Figure 4 can be related to the eye by considering converging lens 21 to be the principal plane of the eye and observation plane 27 to be the retina. The human eye normally has a large amount of chromatic aberration (about 1.0 to 1.2 diopters over the central visual range) so this reduction in chromatic aberration can be significant leading to a noticeable improvement in the eye's visual quality, especially as measured by its contrast sensitivity.

Taken together, Figures 1A-4B illustrate that decreasing ray height decreases both monochromatic and chromatic aberrations at the retina, thus increasing the quality of vision. This can be accomplished by either blocking rays with larger distance from the optical axis by decreasing the pupil diameter or by spreading light from these rays evenly and/or widely across the retina so that more aberrant rays contribute much less light to the central retinal blur circle. Another feature of this effect is that the depth of field is increased as the ray height is decreased as illustrated in Figures 1B, 2B, and 3B.

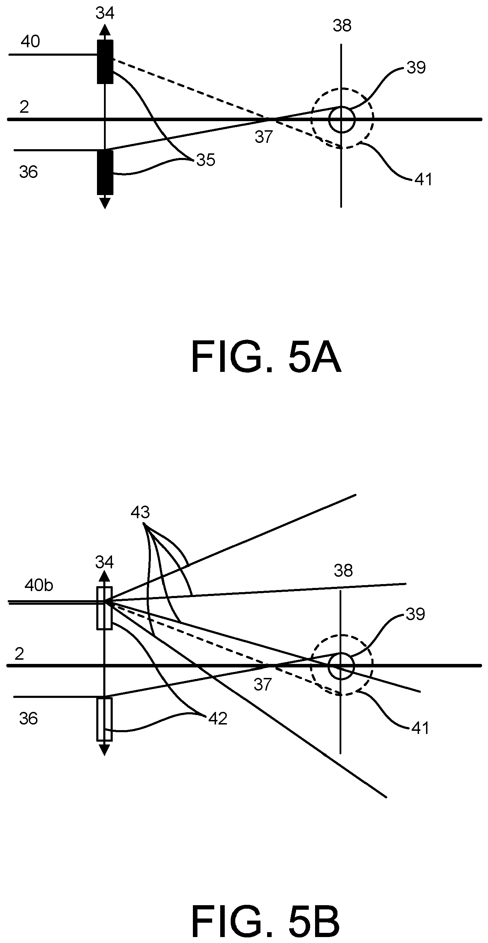

Figure 5A shows a converging lens 34 with optical axis 2 and aperture 35. Incident parallel ray 36 just clears the aperture and thus passes through the lens focal point 37 and intersects the observation plane 38. All parallel rays with the same height as ray 36 trace a small blur circle 39 on the observation plane. Incident parallel ray 40 is blocked by the aperture, and thus it cannot continue to the observation plane to cause a larger blur circle 41. In this way, an aperture which reduces the incident ray height reduces the blur diameter on the observation plane.

Figure 5B illustrates a "virtual aperture". That is, it is not really an aperture that blocks rays, but the optical effect is nearly the same on central vision. In this figure, bundle of rays 40b incident on the virtual aperture propagate through the virtual aperture 42 and through refraction, diffraction, scattering, and/or reflection, yield rays 43 which are widely spread out so there is very little contribution to stray light (blurring light) at any one spot on the observation plane. This is a principal mechanism of operation of the disclosed IOL.

Exemplary Optical Layout of the IOL

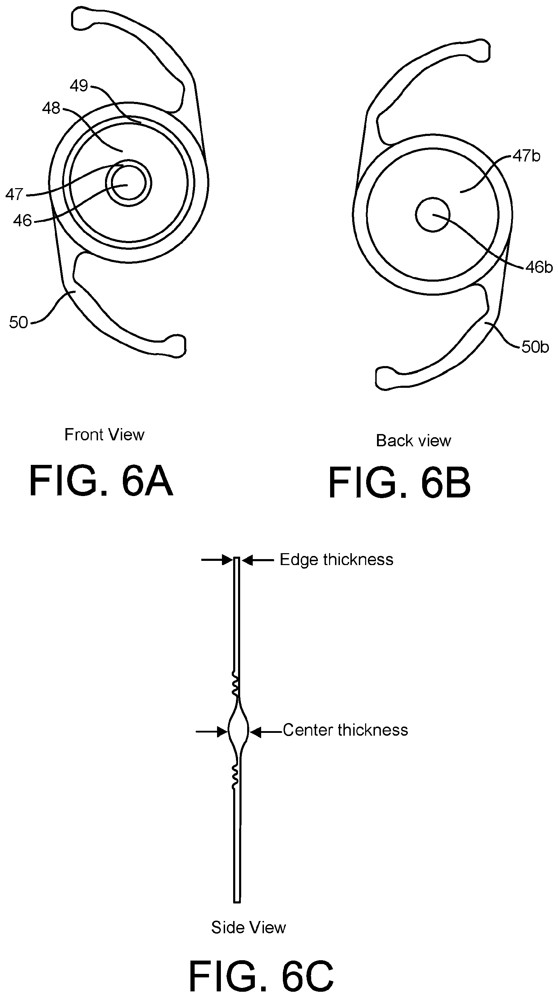

Figures 6A-6C illustrate a layout of an example IOL that employs optical principles to achieve the benefits of decreased monochromatic and chromatic aberrations and increased depth of field. Figure 6A shows a front view of the IOL wherein the front view may be an anterior view. Figure 6B shows a back view of the IOL wherein the back view may be a posterior view. Figure 6C shows a side view of the IOL. The IOL includes a central optical zone 46 (with back side 46b) that provides correction of defocus, astigmatism, and any other correction required of the lens such as spherical aberration. Generally, for an IOL using a virtual aperture, the central optical zone diameter is smaller than that of a traditional IOL. This leads to a smaller central thickness which in turns makes the IOL easier to implant and allows a smaller corneal incision during surgery, such as an incision on the order of 2.2 mm.

The IOL includes a virtual aperture 48 that is positioned further peripherally outward relative to the center location of the central optical zone 46. Moving peripherally outward from the virtual aperture 48, at least one IOL haptic 50 (with back side 50b) is located on the IOL. The haptic 50 can be formed of one or more arms that extend peripherally outward to define a peripheral most edge of the IOL. In an example, the optical zone has a diameter of 1.5mm. The haptic 50 may define an outermost peripheral region of the IOL. A first plurality of light rays incident on an anterior optical surface of the optical zone can pass through the optical zone to form an image on a retina when the IOL is positioned in an eye, while a second plurality of light rays incident on an anterior virtual aperture surface are dispersed widely downstream from the IOL towards and across the retina, such that the image comprises an extended depth-of-field and further wherein the virtual aperture reduces monochromatic and chromatic aberrations in the image. The optical zone can comprise at least one of bifocal optics, trifocal optics and multifocal optics.

The virtual aperture is connected to the optical zone 46 by a first transition region 47, which is located at a peripheral edge of the optical zone 46 such that the virtual aperture is a first periphery region that surrounds or partially surrounds the optical zone. The haptic can comprise a second periphery region for positioning the intraocular lens within an eye. The first transition region is located peripherally outward of the optical zone 46. A second transition region 49 connects the haptic 50 to the virtual aperture 48. The first transition region 47 and the second transition region 49 are configured to ensure zero- and first-order continuity of an outer surface of the IOL on either side of the respective transition region. A common way to implement these transition regions is a polynomial function such as a cubic Bezier function. Transition methods such as these are known to those skilled in the art. On the back side of the IOL is a central optic zone 46b, a haptic 50b, and a transition 47b between them. Figures 6A-6C are not necessarily to scale, and the haptic shape is for illustration purposes only. Other haptic shapes and sizes known to those skilled in the art would be suitable as well. The first and second transition regions are not necessarily present per se in the IOL.

The IOL has an anterior surface and a posterior surface and the components of the IOL including the optical zone 46, the first transition region 47, the second transition region 49, the virtual aperture 48, the haptic 50 can each have a respective anterior surface and posterior surface. The optical zone 46 has an anterior optical surface that can include at least one multifocal zone and/or a toric region. At least a portion or region of the anterior surface and/or the posterior surface, such as in the region of the virtual aperture or other portion of the IOL, can have a surface contour or shape that achieves a desired or predetermined effect for light passing therethrough. In nonlimiting examples, the surface contour of the anterior surface and/or the posterior surface includes a region with a ripple-type contour such as a wave shape or an undulating shape that forms a series of raised and lowered surfaces. The surface contours can achieve various effects with respect to light passing through the IOL. For example, the surface contour can achieve a wide or wider spread of stray light depending upon the type of surface contour used. The surface contour can be used to achieve a spread of stray light which is guided away from a focal point of the retina.

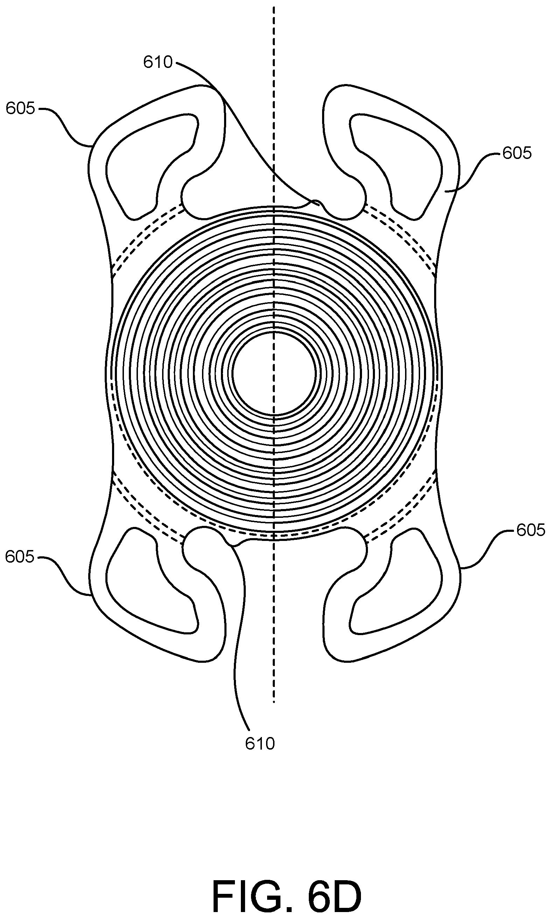

Figure 6D shows a front view of another embodiment of an IOL, which includes a central optical zone, a plurality of peripheral haptics 605, and at least one zone having a surface contour such as a ripple or wave as described further below.

In an example, the optical zone has a diameter of 1.5 mm and serves as a lens which brings distant objects into sharp focus on the central retina.

The IOL includes one or more orientation structures 610 such as one or more protrusions or nubs. In the illustrated embodiment, the orientation structures 610 are positioned on a peripheral edge of a portion of the IOL with at least one orientation structure 610 on the first side of a vertical meridian of the IOL and a second orientation structure 610 on a second side of the vertical meridian. Meridian. The vertical meridian is shown as a dashed line in Figure 6D. The orientation structures 610 are configured to allow a clinician, such as a surgeon, to easily detect that the IOL has a correct side facing the front of the eye. Note that if the IOL were oriented with the back side facing the front of the eye, the orientation structures 610 would be counter-clockwise with respect to the vertical of the lens.

A discussed, the haptic(s) 605 provide a mechanical interface with the eye and holds the various zones of the IOL at its proper position relative to the eye.

Example Optic Zone Details - Hexagonal micro-lens virtual aperture

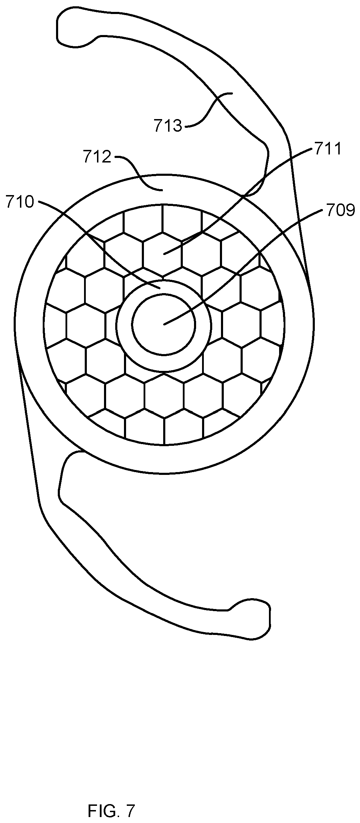

In accordance with the present invention, Figure 7 illustrates a front view of an IOL that includes a virtual aperture having one or more hexagonal structures. The IOL has central optical zone 709, a first transition zone 710, a hexagonal micro-lens virtual aperture 711, a second transition zone 712, and a haptic 713. The first transition zone 710 connects the central optical zone 709 to the hexagonal micro-lens virtual aperture 711 while the second transition zone 712 connects the hexagonal micro-lens virtual aperture 711 to the haptics 713.

The virtual aperture employs a two-dimensional hexagonal sampled array of micro-lenses which mimics the photo sensor sampling of the retina. This arrangement is a beneficial layout for widely spreading light across the retina when the IOL is implanted in an eye.

The hexagonal micro-lens virtual aperture 711 include a plurality of hexagonal shaped microstructures positioned on a front side and/or a backside of the IOL. The hexagonal shape is with respect to an outer boundary of each hexagonal micro-structure has an outer boundary defined by a hexagon microstructure when viewed from a front or rear of the IOL. That is, a hexagonal micro-structure can have an outer boundary defined by a hexagon. A small lens is placed inside the bounds of each of the hexagonal micro-structures. The lens can be a structure that is positioned on or in the micro-structure. The lens may also be monolithically formed as part of the microstructure during manufacture. To help prevent unwanted patterning of light on the retina, the centers of micro-lenses inside each hexagon are randomly moved or positioned on the IOL, and the radii of the micro-lenses are also adjusted. To facilitate manufacturing of the hexagonal micro-lens virtual aperture, between the hexagon boundaries of the micro-lenses, a blending region or fillet is placed with a radius of curvature greater than the radius of a lathe cutter that forms the micro-lens. This radius is on the order of 0.05 mm in a non-limiting example.

The hexagon can have a variety of dimensions. In an embodiment, the hexagon of a micro-structure is more tall than wide. In another embodiment, the hexagon of a micro-structure is more wide than tall. In another embodiment, the outer boundary of a micro-structure is an arbitrarily-shaped polygon.

With reference still to Figure 7, the first transition zone 710 is configured to provide a smooth structural blend between the edge of the optical zone 709 and the central hexagonal micro-lens region 711. The second transition zone 712 is responsible for providing a smooth structural blend between the peripheral hexagonal micro-lens region 711 and the haptic 713. These transition regions can be effectively accomplished using Bezier curves or portions of Bezier surfaces to define a surface of the respective zone. Other transition functions can be suitable as well and are known to those skilled in the art. It should be appreciated that any of the embodiments of the IOLs described herein can be configured to not include any transition zones.

The micro-lenses are implemented as one or more outer surfaces defined at least partially by a sphere, conicoid, or other similar outer surface that can achieve high optical power to widely spread incoming light rays across the retina. For example, the micro-lenses are implemented as one or more outer surfaces defined at least partially by a prismatic or pyramid shape. As an example, in the following discussion there are illustrated embodiments with spherical micro-lenses.

Nominal hexagonal sampling

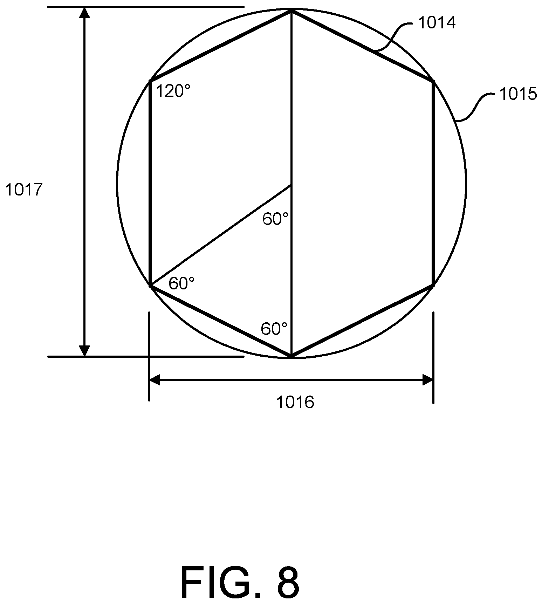

One example hexagon is illustrated in Figure 8, which shows an example micro-structure of the virtual aperture with the micro-structure being defined by a hexagon 1014. In Figure 8, the hexagon 1014 is shown inside a bounding circle 1015 that defines a shape or size of the hexagon. The hexagon has width 1016 and a height 1017. As illustrated, the height of the hexagon is equal to the diameter of the bounding circle 1015. In terms of the radius of the bounding circle, the width of the hexagon is found using Pythagoras's rule to be as given by equation (1) and the height is given by equation (2):

$$width = \sqrt{3} \times r \qquad\qquad (1)$$

$$height = 2 \times r \qquad (2)$$

where

$$r = radius\ of\ bounding\ circle$$

Also, (a) each interior angle of the hexagon is 120 degrees, (b) each side and the center point form an equilateral triangle with interior angle of 60 degrees, and (c) the hexagon side length is equal to the radius of the bounding circle.

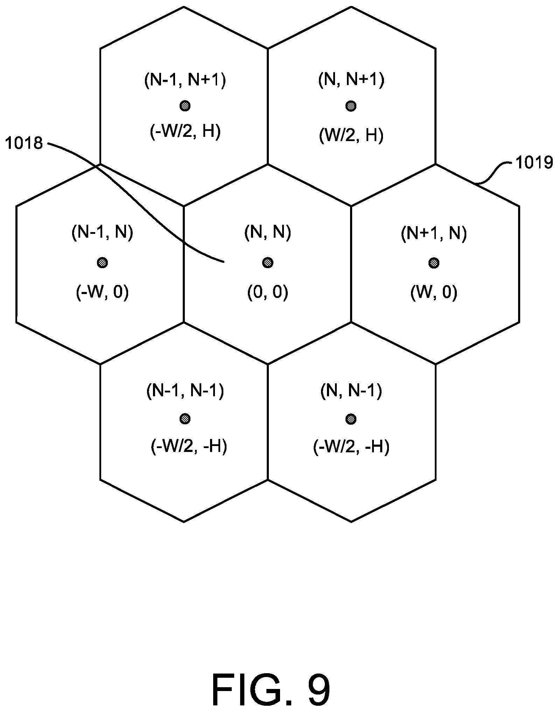

The center portion of a two-dimensional array of hexagons is illustrated in Figure 9. The dimensions of the two-dimensional array of hexagons is defined by equation (3).

$$M\ x\ M = (2N + 1)\ x\ (2N + 1) \qquad (3)$$

In this equation, N is a positive, even integer, for example, 50. The (x, y) location of the center of each hexagon is given by equations (4a) and (4b).

$$x = \begin{cases} (i - N) \times width & for \quad even\ i \\ \left(i - N + \dfrac{1}{2}\right) \times width & for \quad odd\ i \end{cases} \qquad (4a)$$

$$y = (j - N) \times height \qquad (4b)$$

The index of the two-dimensional hexagonal array elements as well as the (x, y) coordinates of the hexagon centers are illustrated in the pairs of values above and below each hexagon center in Figure 9.

Smooth profiles across the micro-lenses

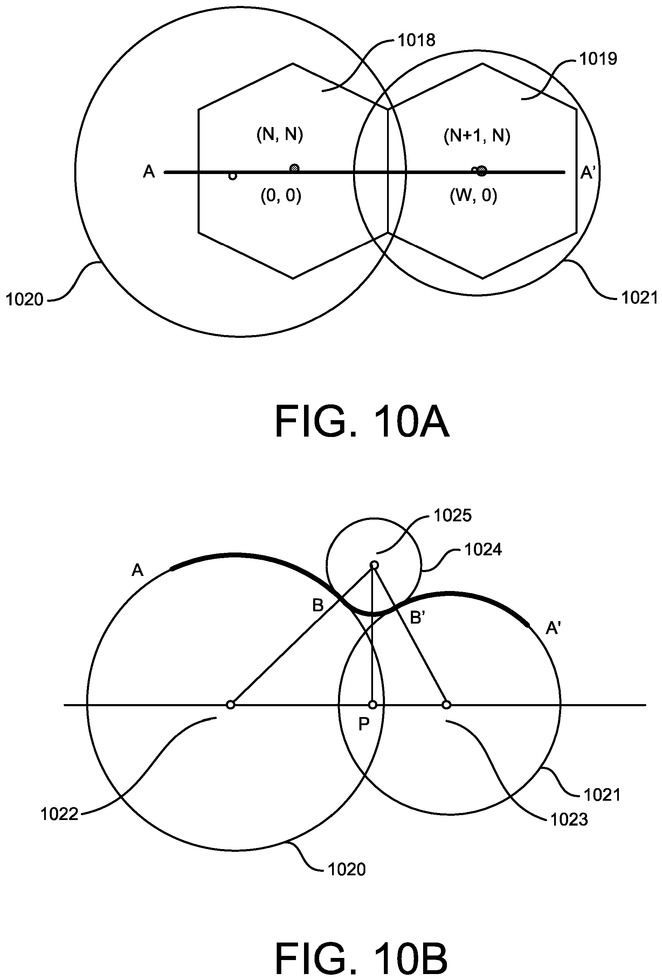

Figure 10A illustrates two, example neighboring or adjacent hexagons at the center of the two-dimensional array. The center of this array can be in coincidence with the optical axis of the IOL. The hexagon 1018 has its center at the center of the optical axis of the IOL. A micro-lens spherical surface 1020 has its center located at a random (x, y) distance from the center of hexagon 1018. A hexagon 1019 is a direct neighbor of hexagon 1018 and a micro-lens spherical surface 1021 has its center located at a random (x,y) distance from the center of hexagon 1019. The radius of micro-lens spherical surface 1020 is larger than the radius of micro-lens spherical surface 1021. In Figure 10A, the coordinates can be referred to as (x,y) with z coming out of the page, x directed to the right and y directed up. Thus, this represents a view looking down onto the surface of the lens and each micro-lens spherical surface is convex, thus producing a local positive high-power surface. Figure 10A also shows is profile AA' which extends through the centers of micro-lens spherical surfaces 1020 and 1021.

Figure 10B illustrates the side view of the geometry shown in Figure 10A. The spheres 1020 and 1021 are shown corresponding to the same spheres in Figure 10A. The centers of the spheres are indicated as points 1022 and 1023, corresponding to spheres 1020 and 1021, respectively. The coordinates in this figure can be referred to as (x,z) with y into the page, x directed to the right and z directed up. Here it can be seen that the profile AA' is a curve on the surface of the micro-lens array due to spheres 1020 and 1021 as well as a spherical fillet 1024.

Pursuant to a manufacturing process of an IOL, the radius of a spherical fillet is selected to be larger than the radius of a lathe cutting tool so that the surface can be generated with the given cutting tool. To find the surface points of the smooth profile AA', the center 1025 of the spherical fillet 1024 is defined as a known radius. For simplicity the centers of the micro-lenses are constrained to have z value on a plane perpendicular to the optical axis. The point P shown in Figure 10B has the same (x,y) coordinates as the center 1025 of fillet sphere 1024 and is located on the line connecting the micro-lens centers 1022 and 1023. The coordinates of the point P are given in equation (5a).

$$\mathbf{P} = \begin{bmatrix} P_x \\ P_y \\ P_z \end{bmatrix} = \begin{bmatrix} x_1 \\ y_1 \\ z_1 \end{bmatrix} + t \begin{bmatrix} a \\ b \\ c \end{bmatrix} \qquad (5a)$$

where,

$$a = x_2 - x_1$$

$$b = y_2 - y_1 \tag{5b}$$

$$c = z_2 - z_1$$

$$t = \frac{d - a\,x_1 - b\,y_1 - c\,z_1}{a^2 + b^2 + c^2} \tag{5c}$$

$$d = \frac{R_1^2 - R_2^2 - (x_1^2 - x_2^2) - (y_1^2 - y_2^2) - (z_1^2 - z_2^2)}{2} \tag{5d}$$

In these equations,

$R_1^2$ = the radius of the first sphere (item 1020) plus the radius of the fillet sphere

$R_2^2$ = the radius of the second sphere (item 1021) plus the radius of the fillet sphere

$x_1, y_1, z_1$ = the center of the first sphere (item 1022)
$x_2, y_2, z_2$ = the center of the first sphere (item 1023)

The set of center points for the center of the fillet sphere for the entire micro-lens spheres can then be found from equation (6a).

$$\begin{bmatrix} x(\theta) \\ y(\theta) \\ z(\theta) \end{bmatrix} = \begin{bmatrix} P_x \\ P_y \\ P_z \end{bmatrix} + r \begin{bmatrix} u_x\,cos(\theta) \\ u_y\,cos(\theta) \\ u_z\,sin(\theta) \end{bmatrix} \tag{6a}$$

where,

$$\mathbf{w} = \frac{\begin{bmatrix} a \\ b \\ c \end{bmatrix}}{\left\| \begin{bmatrix} a \\ b \\ c \end{bmatrix} \right\|}$$

$$\mathbf{v} = \begin{bmatrix} 0 \\ 0 \\ 1 \end{bmatrix} \tag{6b}$$

$$\mathbf{u} = \mathbf{v} \times \mathbf{w}$$

$$r = \sqrt{R_1^2 - (a^2 + b^2 + c^2)t^2}$$

The angle θ is in the plane containing P and perpendicular to the line intersecting the two micro lens sphere centers. Using this geometry, there can be traced out the surface points along the curve segments AB, BB', and B'A'. Together, these points form a continuous blending between each of the micro-lenses in the virtual aperture such that they can be cut on a lathe using a tool of radius less than the fillet sphere radius.

To use the concepts described above to define a surface of the IOL, the following is done. First, the central optic of the IOL is specified such as described in PCT Patent Application Serial No. PCT/US20/37014 and U.S. Patent Application Serial No. 16/380,622. The diameter of the optic zone can be around 1.5 mm and preferably between (1.4 and 1.6 mm) in non-limiting examples. Optical powers for this optic zone vary from -10 to 40 D in steps or 0.25 or 0.5 D. Cylinder powers for toric IOLs vary from 0.5 to 6.0 D in steps of 0.25 to 0.5 D.

The micro-lens array virtual aperture is then generated using the concepts above where the radius of the circles bounding the hexagons is about 0.125 mm. The centers of the individual micro-lens spheres are randomly varied about 0.05 mm in x and y. The radii of the micro-lens spheres are randomly varied 0.05 mm from a mean radius of about 0.2 mm. The width of the virtual aperture region is about 2.0 mm.

The micro-lens array fillet sphere radius is set to be about 25% larger than the lathe tool radius. This can be around 0.05 mm.

The width of the front surface transition regions is each set to around 0.15 mm. The width of the back-surface transition region is set to around 2.3 mm.

The configuration of the haptic is configured according to routine procedures for those skilled in the art.

Once the front and back surfaces have been specified, individual profile samples are taken from the center of the IOL to the periphery to specify the points for the lathe cutting file.

Multi-Region Optical Zone

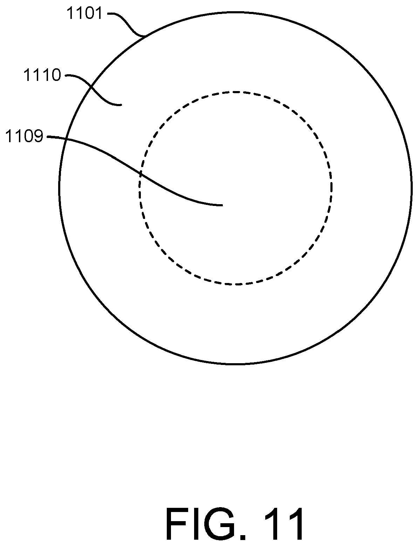

Figure 11 schematically illustrates a multi-region, such as two-region, optical zone 1101 that can be included in any IOL described herein. The regions are indicated 1109 and 1110. These represent two distinct regions in the optical zone for two distinct powers. For example, a first discrete region is a central region 1109 is normally for providing distance vision. A second discrete region is a peripheral region 1110 is normally for providing near vision. The "add" of the near vision region is around 3.0D and in the range of 2.0 to 3.5D.

Due to the special nature of IOL's optical mechanism of action, providing a bifocal optical zone is not as problematic as normal size optical zones of 5.0 mm and larger. This is because the extra aberrations caused by incident rays which are outside the central optical zone diameter of, typically, 1.5 mm, are widely distributed across the retina so as not to negatively affect the central vision of the eye.

Distribution of optic zone regions

In an example configuration, the distance power region of the central optic takes up 75% of the optic zone area and the near power region of the central optic takes up 25% of the optic zone area. Since the diameter of the central optic zone is typically 1.5 mm, the central region 1109 of the optical zone has diameter 1.3 mm and the remainder of the optic zone provides 25% for the near vision region 1110.

For some eyes it can be preferred to have the distribution of distance region area and near region area portioned to 50% each or 25% for distance and 75% for near vision. Providing one eye with a majority of the optic zone area for distance vision, such as 75 to 100%, and the other eye with more area optic zone area for near vision may would be used for extended depth of focus / monovision patients. In this case, both eyes have extended depth of focus, but one eye (usually the dominant eye) has slightly better performance for distance vision and the other eye has slightly better visual performance for near vision.

Optic surfaces for the optic zone regions

To provide the desired optical powers for the optic zone regions, either conic refractive profiles can be used, or diffractive profiles can be used.

In the case of simple conic refractive profiles, each optic zone provides its optical power via a conic curve such that the apical radius of curvature provides the desired optical power and the conicity (K) value is set to reduce spherical aberrations for the region. Optimization to find the apical radius and the conicity can be done numerically using commercially available optical design programs such as Zemax or using closed form analytical equations. Both of these methods are known to those skilled in the art.

When simple conic refractive profiles are used and the central region 9 of the optical zone provides distance vision and the peripheral region 10 provides near vision, the transition between the regions is negligibly small. This is the preferred arrangement as transition regions generally cause stray light that would otherwise be properly focused by one of the two optical power regions.

When simple conic refractive profiles are used and the central region 9 of the optical zone provides near vision and the peripheral region 10 provides distance vision, the transition between the regions is required to smoothly join the regions. This transition profile is generally implemented by either a Bezier curve or a circular fillet, both of which are known to those skilled in the art.

Peripheral add zone

In another embodiment the add zone can be placed inside the virtual aperture region. In still another embodiment the add zone can be placed on the posterior side inside the large transition region. The peripheral add zone could be present along with the add zone in the central optic.

Cylinder power to correct astigmatism

To correct for astigmatism, a cylinder component can be added to one or both surfaces of the IOL optic zone. The cylinder power for this purpose is in the range of 0.5 to 6.0 diopters in steps of either 0.25 or 0.5 D.
To use the concepts described above to define a surface of the IOL, the following is done. First, the central optic of the IOL is specified as explained above. The diameter of the optic zone is around 1.5 mm and preferably between (1.4 and 1.6 mm). Optical powers for this optic zone vary from -10 to 40 D in steps or 0.25 or 0.5 D. Cylinder powers for toric IOLs vary from 0.5 to 6.0 D in steps of 0.25 to 0.5 D.
The virtual aperture is then generated using the concepts described in previous disclosures. The width of the virtual aperture region is about 2.0 mm.
The width of the front surface transition regions is each set to around 0.15 mm. The width of the back-surface transition region is set to around 2.3 mm.
The design of the haptic is considered a separate issue and is routine for those skilled in the art.
Once the front and back surfaces have been specified, individual profile samples are taken from the center of the IOL to the periphery to specify the points for the lathe cutting file.
While this specification contains many specifics, these should not be construed as limitations on the scope of an invention that is defined in the appended claims but rather as descriptions of features specific to particular embodiments. Certain features that are described in this specification in the context of separate embodiments can also be implemented in combination in a single embodiment.

**Claims**

1.    An intraocular lens configured to provide an extended depth-of-field, said intraocular lens comprising:

an optical zone (709; 1101) comprising at least one anterior optical surface and at least one posterior optical surface;
a first periphery region peripherally positioned relative to the optical zone (709); and
a second periphery region peripherally positioned relative to the first periphery region, the second periphery region comprising a haptic ( 713) for positioning the intraocular lens within an eye, wherein the haptic (713) comprises an outermost region of the intraocular lens;
wherein light rays incident on the anterior optical surface pass through the optical zone (709; 1101) to form an image on a retina when the intraocular lens is implanted in an eye; and
**characterized in that** the first periphery region comprises a virtual aperture (711), the virtual aperture (711) comprising an anterior virtual aperture surface and a posterior virtual aperture surface, wherein the virtual aperture comprises a plurality of hexagonal micro-structures (1014, 1018, 1019), each hexagonal micro-structure (1014, 1018, 1019) comprising a micro-lens;
wherein light rays incident on the anterior virtual aperture surface are dispersed widely downstream from the intraocular lens towards and across the retina by the plurality of hexagonal micro-structures (1014, 1018, 1019), such that the image comprises the extended depth-of-field and further wherein said virtual aperture reduces monochromatic and chromatic aberrations in the image.

2.    The intraocular lens of claim 1, wherein each hexagonal micro-structure (1014, 1018, 1019) has an outer boundary defined by a hexagon.

3.    The intraocular lens of claim 1, wherein at least one micro-lens comprises a sphere.

4. The intraocular lens of claim 1, wherein at least one micro-lens comprises a conicoid.

5. The intraocular lens of claim 1, wherein the first periphery region is connected to the central optical zone by a first transition region ( 710).

6. The intraocular lens of claim 1, wherein the second periphery region is connected to the first periphery region by a second transition region (710).

7. The intraocular lens of claim 1, wherein the optical zone (709; 1101) includes at least two discrete regions including a first discrete region (1109) and a second discrete region (1110).

8. The intraocular lens of claim 7, wherein the first discrete region (1109) is a central region and the second discrete region (1110) is a peripheral region positioned peripherally around the central region (1109).

9. The intraocular lens of claim 7, wherein the first discrete region (1109) comprises a first distance power and the second discrete region (1110) comprises a second distance power.

**Patentansprüche**

1. Intraokulare Linse, die dafür ausgelegt ist, eine erweiterte Schärfentiefe bereitzustellen, wobei die intraokulare Linse aufweist:

eine optische Zone (709; 1101), die mindestens eine vordere optische Oberfläche und mindestens eine hintere optische Oberfläche aufweist;
einen ersten peripheren Bereich, der umlaufend relativ zur optischen Zone (709) positioniert ist; und
einen zweiten peripheren Bereich, der umlaufend relativ zum ersten peripheren Bereich positioniert ist, wobei der zweite periphere Bereich eine Haptik (713) zum Positionieren der intraokularen Linse in einem Auge aufweist, wobei die Haptik (713) einen am weitesten außen liegenden Bereich der intraokularen Linse aufweist;
wobei Lichtstrahlen, die auf die vordere optische Oberfläche auftreffen, durch die optische Zone (709, 1101) hindurchgelangen, um ein Bild auf einer Retina zu bilden, wenn die intraokulare Linse in ein Auge implantiert ist; und
**dadurch gekennzeichnet, dass** der erste periphere Bereich eine virtuelle Apertur (711) aufweist, wobei die virtuelle Apertur (711) eine vordere virtuelle Aperturoberfläche und eine hintere virtuelle Aperturoberfläche aufweist, wobei die virtuelle Apertur eine Vielzahl von hexagonalen Mikrostrukturen (1014, 1018, 1019) aufweist, wobei jede hexagonale Mikrostruktur (1014, 1018, 1019) eine Mikrolinse aufweist;
wobei Lichtstrahlen, die auf die vordere virtuelle Aperturoberfläche auftreffen, durch die Vielzahl von hexagonalen Mikrostrukturen (1014, 1018, 1019), stromabwärts der intraokularen Linse in Richtung zur und über die Retina hinweg weit gestreut werden, so dass das Bild die erweiterte Schärfentiefe aufweist, und darüber hinaus wobei die virtuelle Apertur monochromatische und chromatische Aberrationen in dem Bild verringert.

2. Intraokulare Linse nach Anspruch 1, wobei jede hexagonale Mikrostruktur (1014, 1018 1019) eine Außenumrandung hat, die durch ein Hexagon definiert ist.

3. Intraokulare Linse nach Anspruch 1, wobei mindestens eine Mikrolinse eine Kugeloberfläche aufweist.

4. Intraokulare Linse nach Anspruch **1,** wobei mindestens eine Mikrolinse einen Rotationskegel aufweist.

5. Intraokulare Linse nach Anspruch **1,** wobei der erste periphere Bereich durch einen ersten Übergangsbereich (710) mit der zentralen optische Zone verbunden ist.

6. Intraokulare Linse nach Anspruch **1,** wobei der zweite periphere Bereich durch einen zweiten Übergangsbereich (710) mit dem ersten peripheren Bereich verbunden ist.

7. Intraokulare Linse nach Anspruch **1,** wobei die optische Zone (709; 1101) mindestens zwei diskrete Bereiche aufweist, die einen ersten diskreten Bereich (1109) und einen zweiten diskreten Bereich (1110) umfassen.

8. Intraokulare Linse nach Anspruch 7, wobei der erste diskrete Bereich (1109) ein zentraler Bereich und der zweite diskrete Bereich (1110) ein Umfangsbereich ist, der umlaufend um den zentralen Bereich (1109) herum positioniert ist.

**9.** Intraokulare Linse nach Anspruch 7, wobei der erste diskrete Bereich (1109) eine erste Fernstärke und der zweite diskrete Bereich (1110) eine zweite Fernstärke aufweist.

**Revendications**

**1.** Lentille intraoculaire conçue pour fournir une profondeur de champ étendue, la lentille intraoculaire comprenant :

une zone optique (709 ; 1101) comprenant au moins une surface optique avant et au moins une surface optique arrière ;
une première région périphérique positionnée de manière circonférentielle par rapport à la zone optique (709) ; et
une deuxième région périphérique positionnée de manière circonférentielle par rapport à la première région périphérique, la deuxième région périphérique comprenant une haptique (713) pour positionner la lentille intraoculaire dans un œil, l'haptique (713) comprenant une région la plus externe de la lentille intraoculaire ;
sachant que des rayons lumineux frappant la surface optique avant traversent la zone optique (709, 1101) pour former une image sur une rétine lorsque la lentille intraoculaire est implantée dans un œil ; et
**caractérisée en ce que** la première région périphérique comprend une ouverture virtuelle (711), l'ouverture virtuelle (711) comprenant une surface d'ouverture virtuelle avant et une surface d'ouverture virtuelle arrière, l'ouverture virtuelle comprenant une pluralité de microstructures hexagonales (1014, 1018, 1019), chaque microstructure hexagonale (1014, 1018, 1019) comprenant une microlentille ;
sachant que des rayons lumineux frappant la surface d'ouverture virtuelle avant sont largement diffusés en aval de la lentille intraoculaire vers et au-delà de la rétine à l'aide de la pluralité de microstructures hexagonales (1014, 1018, 1019), de sorte que l'image présente une profondeur de champ étendue, et en outre l'ouverture virtuelle réduit les aberrations monochromatiques et chromatiques dans l'image.

**2.** La lentille intraoculaire selon la revendication 1, sachant que chaque microstructure hexagonale (1014, 1018, 1019) a un contour extérieur défini par un hexagone.

**3.** La lentille intraoculaire selon la revendication 1, sachant qu'au moins une microlentille présente une surface sphérique.

**4.** La lentille intraoculaire selon la revendication 1, sachant qu'au moins une microlentille présente un cône de révolution.

**5.** La lentille intraoculaire selon la revendication 1, sachant que la première région périphérique est reliée à la zone optique centrale par une première zone de transition (710).

**6.** La lentille intraoculaire selon la revendication 1, sachant que la deuxième région périphérique est reliée à la première région périphérique par une deuxième zone de transition (710).

**7.** La lentille intraoculaire selon la revendication 1, sachant que la zone optique (709 ; 1101) comprend au moins deux régions discrètes comprenant une première région discrète (1109) et une deuxième région discrète (1110).

**8.** La lentille intraoculaire selon la revendication 7, sachant que la première région discrète (1109) est une région centrale et que la deuxième région discrète (1110) est une zone périphérique positionnée autour de la région centrale (1109).

**9.** La lentille intraoculaire selon la revendication 7, sachant que la première région discrète (1109) présente une première puissance de vision de loin et que la deuxième région discrète (1110) présente une deuxième puissance de vision de loin.

FIG. 1A

FIG. 1B

# FIG. 2A

# FIG. 2B

# FIG. 3A

# FIG. 3B

# FIG. 4A

# FIG. 4B

## FIG. 5A

## FIG. 5B

Front View

# FIG. 6A

Back view

# FIG. 6B

Edge thickness

Center thickness

Side View

# FIG. 6C

FIG. 6D

FIG. 7

FIG. 8

**FIG. 9**

FIG. 10A

FIG. 10B

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62951832
- US 62951839
- US 63056110
- US 2005068494 A1
- US 10285807 B
- US 380622
- US 2037014 W